# EUROPÄISCHE PATENTSCHRIFT

(11) **EP 2 046 813 B1**
(45) Veröffentlichungstag und Bekanntmachung des Hinweises auf die Patenterteilung: **24.02.2010**
(21) Anmeldenummer: 07786340.5
(22) Anmeldetag: 25.07.2007
(51) Int. Cl.: C07K 5/10, C07K 7/06, A61K 47/48

(54) **PROTEINBINDENDE METHOTREXAT-DERIVATE UND DIESE ENTHALTENDE ARZNEIMITTEL**
PROTEIN-BINDING METHOTREXATE DERIVATIVES, AND MEDICAMENTS CONTAINING THE SAME
DÉRIVÉS DE MÉTHOTREXATE DE LIAISON PROTÉIQUE ET MÉDICAMENTS CONTENANT LESDITS DÉRIVÉS

(30) Priorität: 28.07.2006 DE 102006035083
(43) Veröffentlichungstag der Anmeldung: 15.04.2009
(73) Patentinhaber: medac Gesellschaft für klinische Spezialpräparate mbH, 22880 Wedel (DE)
(72) Erfinder: KRATZ, Felix, 79238 Ehrenkirchen (DE); WARNECKE, André, 79117 Freiburg (DE)
(74) Vertreter: Müller-Boré & Partner Patentanwälte
(86) Internationale Anmeldenummer: PCT/EP2007/006618
(87) Internationale Veröffentlichungsnummer: WO 2008/012086

(56) Entgegenhaltungen:
- WO-A-2005/085294
- FITZPATRICK J J ET AL: "DESIGN, SYNTHESIS AND IN VITRO TESTING OF METHOTREXATE CARRIER CONJUGATES LINKED VIA OLIGOPEPTIDE SPACERS" ANTI-CANCER DRUG DESIGN, BASINGSTOKE, GB, Bd. 10, Nr. 1, Januar 1995 (1995-01), Seiten 1-9, XP001053201 ISSN: 0266-9536
- UMEMOTO N ET AL: "MOLECULAR DESIGN OF METHOTREXATE-ANTIBODY CONJUGATES FOR TARGETED CANCER TREATMENT" JOURNAL OF BIOACTIVE AND COMPATIBLE POLYMERS, LANCASTER, PA, US, Bd. 7, Nr. 2, April 1992 (1992-04), Seiten 191-219, XP009020458
- CHAU Y ET AL: "SYNTHESIS AND CHARACTERIZATION OF DEXTRAN-PEPTIDE-METHOTREXATE CONJUGATES FOR TUMOR TARGETING VIA MEDIATION BY MATRIX METALLOPROTEINASE II AND MATRIX METALLOPROTEINASE IX" BIOCONJUGATE CHEMISTRY, ACS, WASHINGTON, DC, US, Bd. 15, Nr. 4, Juli 2004 (2004-07), Seiten 931-941, XP001236871 ISSN: 1043-1802
- CHAU YING ET AL: "Investigation of targeting mechanism of new dextran-peptide-methotre xate conjugates using biodistribution study in matrix-metalloproteinase-overexpressing tumor xenograft model." JOURNAL OF PHARMACEUTICAL SCIENCES MAR 2006, Bd. 95, Nr. 3, März 2006 (2006-03), Seiten 542-551, XP002464217 ISSN: 0022-3549
- KRATZ F: "Drug conjugates with albumin and transferrin" EXPERT OPINION ON THERAPEUTIC PATENTS, ASHLEY PUBLICATIONS, GB, Bd. 12, Nr. 3, 2002, Seiten 433-439, XP002337252 ISSN: 1354-3776

## Beschreibung

Die vorliegende Erfindung betrifft Methotrexat-Derivate bzw. Methotrexat-PeptidDerivate, die eine proteinbindende Gruppe enthalten und enzymatisch im Körper unter Freisetzung des Wirkstoffs oder eines niedermolekularen Wirkstoff-Derivates spaltbar sind, ein Verfahren zur Herstellung von Methotrexat-Derivaten, deren Verwendung und Methotrexat-Derivate umfassende Arzneimittel.

Methotrexat (MTX) ist ein Folsäureantagonist, der bei der Behandlung von Tumorerkrankungen sowie der rheumatoiden Arthritis eingesetzt wird. Sein Einsatz wird durch eine Reihe von Nebenwirkungen limitiert (z. B. Schwindel, Alopezie, Stomatitis, Magen-Darm-Symptome, erhöhte Infektanfälligkeit). Um das Nebenwirkungsprofil und die Wirksamkeit von MTX bzw. MTX-Derivaten zu verbessern, wurden makromolekulare Transportformen von MTX durch Kopplung des Wirkstoffs an synthetische Polymere wie Poly(ethylenglykol) (Riebeseel, K.; Biedermann, E.; Löser, R.; Breiter, N.; Hanselmann, R. et al. Bioconjugate Chem. 2002, 13, 773-785), HPMA-Copolymere (Subr, V.; Strohalm, J. et al. Controlled Release 1997, 49, 123-132) oder an, gegebenenfalls über bestimmte Peptidspacer gebundenes humanes Serumalbumin (HSA) (Wunder, A.; Muller-Ladner et al. J Immunol 2003, 170, 4793-4801; Wunder, A.; Stehle, G. et al. Int. J. Oncol. 1997, 11, 497-507 i Fitzpatrick, J. et al. Anti-Cancer Drug Design 1995, 10, 1-9) bereitgestellt. Jedoch besteht nach wie vor ein Bedarf für neue MTX bzw. MTX-enthaltende Systeme mit geringem Nebenwirkungsprofil und im Wesentlichen verbesserter Wirksamkeit im Vergleich zu freiem MTX.

Somit liegt der Erfindung die Aufgabe zugrunde, Prodrugs von Methotrexat bereitzustellen, die MTX oder MTX-Derivate in Tumorgewebe oder rheumatisch erkranktem Gewebe freisetzen.

Diese Aufgabe wird durch die in den Ansprüchen gekennzeichneten Ausführungsformen gelöst.

Insbesondere werden Methotrexat-Derivate der allgemeinen Formel 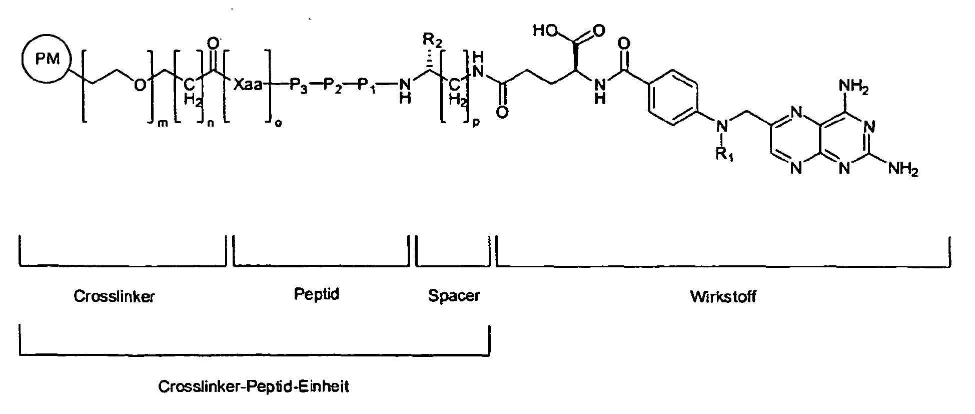 bereitgestellt, worin R₁ = H oder CH₃, R₂ = H oder COOH, P₁-P₃ = L- oder D-Aminosäuren, Xₐₐ eine löslichkeitsvermittelnde Aminosäure, m = 0 bis 6, n = 0 bis 5, o = 0 bis 2, p = 1 bis 10 bedeuten und PM eine proteinbindende Gruppe ist.

Gemäß der vorliegenden Erfindung erlaubt eine integrierte hydrolytisch oder enzymatisch spaltbare Sollbruchstelle hierbei eine kontrollierte Freisetzung des Wirkstoffs oder eines Spacer-Wirkstoff-Derivates *in vivo,* sodass Methotrexat-Derivate der vorliegenden Erfindung Prodrugs darstellen.

Die erfindungsgemäßen MTX-Derivate sind aus einer antitumoral bzw. antirheumatisch wirksamen Methotrexat-Komponente, einem Spacermolekül, einer Peptidkette und einem heterobifunktionellen Crosslinker aufgebaut. Im Folgenden wird dieser Aufbau näher erläutert:

Die antitumoral wirksame MTX-Komponente gemäß der vorliegenden Erfindung ist ein Wirkstoff der allgemeinen Formel 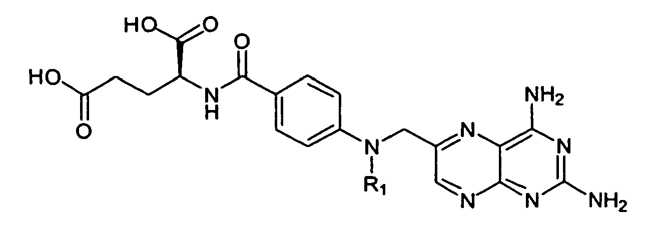 worin
R₁ = CH₃ oder H
bedeutet. Bevorzugter Wirkstoff ist Methotrexat.

Das Spacermolekül gemäß der vorliegenden Erfindung ist ein Diamin der allgemeinen Formel 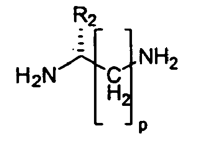 worin
R₂ = H oder COOH
p = 1 bis 10
bedeuten.

Bevorzugte Spacer sind Ethylendiamin (R₂ = H, p = 1) und Spacer, bei denen p = 4 oder 5 ist. Ein besonders bevorzugter Spacer ist _{L}-Lysin (R₂ = COOH, p = 4).

In der vorliegenden Erfindung setzt sich das Peptid aus einer enzymatisch spaltbaren Sequenz sowie einer *N*-terminalen löslichkeitsvermittelnden Komponente zusammen und hat die allgemeine Formel 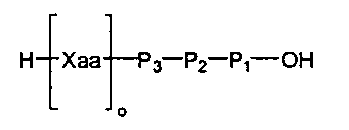 worin
P₁-P₃ = L- oder D-Aminosäuren
Xₐₐ = eine Aminosäure mit basischer Seitenkette
o = 0-2
bedeuten.

In der vorliegenden Erfindung ist die Aminosäure P₁ ausgewählt unter den Aminosäuren Lysin, Methionin, Alanin, Prolin und Glycin. Die Aminosäure P₂ ist ausgewählt unter den Aminosäuren Leucin, Phenylalanin, Methionin, Alanin, Prolin und Tyrosin. Die Aminosäure P₃ ist ausgewählt unter den Aminosäuren D-Alanin, Alanin, _{D}-Valin, Valin, Leucin und Phenylalanin. Bevorzugte Aminosäuren in der P₁-Position sind Lysin, Alanin und Methionin. Bevorzugte Aminosäuren in der P₂-Position sind Phenylalanin, Methionin, Alanin und Tyrosin. Bevorzugte Aminosäuren in der P₃-Position sind _{D}-Alanin, Alanin, _{D}-Valin, Valin und Phenylalanin.

Besonders bevorzugte Peptidsequenzen sind in der untenstehenden Tabelle abgebildet.

| **P₃** | **P₂** | **P₁** |
|---|---|---|
| D-Ala | Phe | Lys |
| Ala | Phe | Lys |
| D-Val | Leu | Lys |
| Val | Leu | Lys |
| Ala | Phe | Met |
| Phe | Ala | Met |
| Ala | Met | Met |
| Phe | Met | Met |

Gemäß der vorliegenden Erfindung ist die löslichkeitsvermittelnde Gruppe Xaa bevorzugt ausgewählt unter den Aminosäuren Arginin, Lysin und Histidin. Eine besonders bevorzugte Gruppe ist Arginin.

In der vorliegenden Erfindung ist der heterobifunktionelle Crosslinker eine Carbonsäure mit einer proteinbindenden Gruppe der allgemeinen Formel 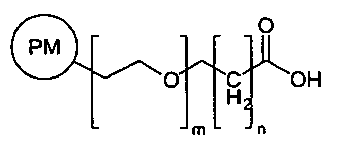 worin
m = 0 bis 6
n = 0 bis 5
PM = proteinbindende Gruppe
bedeuten.

Die proteinbindende Gruppe (PM) ist bevorzugt ausgewählt unter einer 2-Dithiopyridylgruppe, einer Halogenacetamidgruppe, einer Halogenacetatgruppe, einer Disulfidgruppe, einer Acrylsäureestergruppe, einer Monoalkylmaleinsäureestergruppe, einer Monoalkylmaleaminsäureamidgruppe, einer *N*-Hydroxysuccinimidylestergruppe, einer Isothiocyanatgruppe, einer Aziridingruppe oder einer Maleinimidgruppe. Eine besonders bevorzugte proteinbindende Gruppe ist die Maleinimidgruppe.

Bevorzugte Crosslinker sind durch m = 3 und n = 1 sowie durch m = 0 und n = 4 gekennzeichnet.

Gemäß der vorliegenden Erfindung sind Wirkstoff und Spacermolekül durch eine
Amidbindung zwischen der γ-Carboxylgruppe des Wirkstoffs und der ersten Aminogruppe des Spacermoleküls verbunden. Die Bindung zwischen Spacermolekül und der Crosslinker-Peptid-Einheit besteht aus einer Amidbindung zwischen der zweiten Aminogruppe des Spacermoleküls und der C-terminalen Carboxylgruppe der Crosslinker-Peptid-Einheit. Die Bindung zwischen Crosslinker und der Peptidkette besteht aus einer Amidbindung zwischen dem N-Terminus der Peptidkette und der Carboxylgruppe des Crosslinkers.

Eine wesentliche Eigenschaft der erfindungsgemäßen MTX-Derivate besteht darin, dass die Bindung zwischen Spacermolekül und Crosslinker enzymatisch spaltbar ist, wodurch eine kontrollierte Freisetzung des Wirkstoffs oder eines Spacer-Wirkstoff-Derivates in Tumorgewebe und rheumatisch-erkranktem Gewebe ermöglicht wird. Proteasen wie z. B. Cathepsine oder Plasmin werden in vielen humanen Tumoren und rheumatischem Gewebe überexprimiert, wodurch sie einen idealen Angriffspunkt für eine Target-orientierte, enzymatische Aktivierung von Prodrugs darstellen (Yan, S. et al. Biol. Chem. 1998, 2, 113-123; Leto, G. et al. Clin. Exp. Metastasis 2004, 91-106; Sloane, B..F.; Yan, S. et al. Seminars in Cancer Biology, 2005, 15, 149-157; Dano, K.; Behrendt, N. et al. Thrombosis & Haemostasis 2005, 93, 676-681; Hashimoto, Y.; Kakegawa, H. et al. Biochem. Biophys. Res. Commun. 2001, 283, 334-339; Ikeda, Y.; Ikata, T. et al. J. Med. Invest. 2000, 47, 61-75). Des Weiteren zeigen die erfindungsgemäßen MTX-Derivate eine rasche Spaltung in experimentellen Tumorhomogenaten und Synovialflüssigkeiten von Patienten mit rheumatoider Arthritis (siehe Beispiele 4 und 5).

Die Herstellung der erfindungsgemäßen MTX-Derivate erfolgt bevorzugt durch Kondensation von Methotrexat-Derivaten der allgemeinen Formel 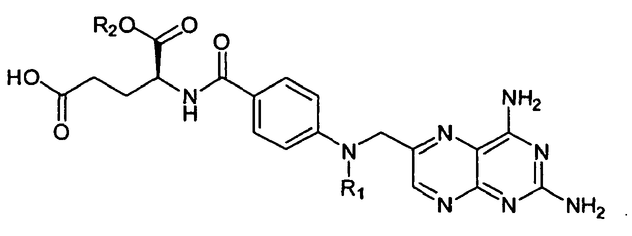 worin
R₁ = CH₃, H oder COCF₃
R₂ = C(CH₃)₃, eine Alkoxy-substituierte Benzylgruppe oder eine Trialkylsilylgruppe bedeuten, mit einer Crosslinker-Peptid-Einheit der allgemeinen Formel 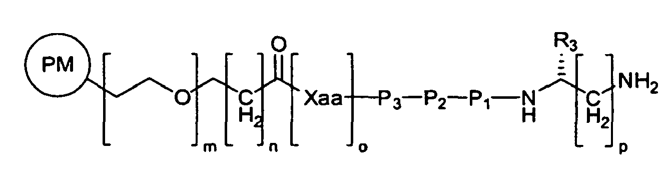 worin
R₃ = H, COOH oder COOtBu
P₁ = Lysin, Methionin, Alanin, Prolin oder Glycin
P₂ = Leucin, Phenylalanin, Methionin, Alanin, Prolin oder Tyrosin
P₃ = D-Alanin, Alanin, D-Valin, Valin, Leucin oder Phenylalanin
Xₐₐ = Aminosäure mit basischer Seitenkette
m = 0 bis 6
n = 0 bis 5
o = 0 bis 2
p = 1 bis 10
PM eine proteinbindende Gruppe
bedeuten, wobei etwaige nukleophile Gruppen an P₁, P₂ und Xaa gegebenenfalls durch dem Fachmann geläufige Schutzgruppen geschützt vorliegen.

Dabei werden gemäß der vorliegenden Erfindung als Reagenzien zur Aktivierung der Carboxylgruppe der Crosslinker-Peptid-Einheit vorzugsweise O-(Azabenzotriazol-1-yl)-*N,N,N*'*,N*'-tetramethyluronium-Hexafluorophosphat (HATU), (Benzotriazol-1-yloxy)tris(dimethylamino)phosphonium-Hexafluorophosphat (BOP), *N*,*N*'-Diiopropylcarbodiimid (DIPC), *N*,*N*'-Dicyclohexylcarbodiimid (DCC) oder 2-Chlor-1-methylpyridiniumiodid unter Zusatz gängiger Katalysatoren bzw. Hilfsbasen wie z. B. *N*-Ethyldiiopropylamin (DIEA), Trialkylamine, Pyridin, 4-Dimethylaminopyridin (DMAP) oder Hydroxybenzotriazol (HOBt) eingesetzt. Die Umsetzung erfolgt beispielsweise in einem polaren organischen Lösungsmittel, vorzugsweise in *N,N*-Dimethylformamid. Die Umsetzungen werden beispielsweise bei Temperaturen zwischen -10 °C und Raumtemperatur durchgeführt, wobei die Reaktionszeit beispielsweise zwischen 30 Minuten und 48 Stunden liegt. Die Isolierung des Zwischenproduktes erfolgt beispielsweise durch Fällung aus einem unpolaren Lösungsmittel, bevorzugt Diethylether.

In einem darauf folgenden, zweiten Syntheseschritt wird erfindungsgemäß die Schutzgruppe R₂ zusammen mit etwaigen Schutzgruppen für nukleophile Gruppen an P₁, P₂ und Xaa entfernt. Diese Abspaltung wird typischerweise durch Behandlung mit einer Säure, bevorzugt Trifluoressigsäure oder Chlorwasserstoff, erreicht. In einer für das Verfahren bevorzugten Ausführungsweise wird das Produkt des ersten Syntheseschrittes etwa 30 min lang mit einem Gemisch aus Trifluoressigsäure und Dichlormethan im Verhältnis 1:1 behandelt. Durch Fällung aus einem unpolaren Lösungsmittel, bevorzugt Diethylether, wird das Rohprodukt isoliert.

Die Reinigung des Rohproduktes erfolgt gemäß der vorliegenden Erfindung beispielsweise durch Kristallisation oder Säulenchromatographie, bevorzugt an Reverse-Phase-Kieselgel.

Nach einer bevorzugten Ausführungsform der vorliegenden Erfindung wird Methotrexat-γ-*tert*.-butylester mit EMC-D-Ala-Phe-Lys(Boc)-Lys-OH (EMC = 6-Maleinimidocapronsäure) unter Verwendung von HATU als Kopplungsreagenz kondensiert und anschließend mit Trifluoressigsäure behandelt (siehe Beispiel 1).

Die erfindungsgemäßen proteinbindenden Methotrexat-Derivate können parenteral, bevorzugt intravenös appliziert werden. Dazu werden die erfindungsgemäßen MTX-Derivate als Lösungen, Feststoffe oder Lyophilisate, gegebenenfalls unter Verwendung üblicher pharmazeutisch verträglicher bzw. annehmbarer Hilfsstoffe wie Träger oder Verdünnungsmittel bzw. Lösungsmittel bereitgestellt. Solche Hilfsstoffe sind beispielsweise Polysorbate, Glucose, Lactose, Mannitol, Dextrane, Zitronensäure, Tromethamol, Triethanolamin, Aminoessigsäure oder synthetische Polymere oder Gemische davon. Bevorzugt werden die erfindungsgemäßen MTX-Derivate in einem isotonischen Puffer gelöst appliziert. Die Löslichkeit des MTX-Derivates kann gegebenenfalls durch pharmazeutisch verträgliche bzw. annehmbare Lösungsmittel wie etwa 1,2-Propandiol, Ethanol, Isopropanol, Glycerol oder Poly(ethylenglykol) mit einem Molekulargewicht von 200 bis 600 g/mol oder Gemische davon, bevorzugt Poly(ethylenglykol) mit einem Molekulargewicht von 600 g/mol, oder Löslichkeitsvermittler, wie z. B. Tween 80, Cremophor oder Polyvinylpyrrolidon, oder Gemische davon verbessert werden.

Ein wesentliches Merkmal der erfindungsgemäßen MTX-Derivate liegt in einer raschen kovalenten Bindung an Serumproteine über die proteinbindende Gruppe, wodurch eine makromolekulare Transportform des Wirkstoffs generiert wird. Von Serumproteinen wie Transferrin, Albumin und LDL ist eine erhöhte Aufnahme in Tumorgewebe bzw. eine Anreicherung in rheumatisch erkranktem Gewebe bekannt (Kratz F.; Beyer U. Drug Delivery 1998, 5, 281-299; Adams, B. K.; Al Attia, H. M. et al. Nuclear Med. Commun. 2001, 22, 315-318; Sahin, M.; Bernay, I. et al. Ann. Nuclear Med. 1999, 13, 389-395; Liberatore, M.; Clemente, M. et al. J. Nuclear Med. 1992, 19, 853-857), sodass diese im Rahmen der Erfindung als endogene Träger für Zytostatika herangezogen werden können. Ein besonders bevorzugtes Serumprotein ist zirkulierendes Humanserumalbumin (HSA), das mit einer durchschnittlichen Konzentration von 30 bis 50 g/L die Hauptprotein-Komponente des menschlichen Blutes bildet (Peters T. *Adv. Protein Chem.* **1985,** *37*, 161-245) und eine freie Cysteingruppe (Cystein-34-Gruppe) an der Oberfläche des Proteins aufweist, welche zur Anbindung von thiolbindenden Gruppen wie Maleinimiden oder Disulfiden geeignet ist (WO 00/76551). Dass erfindungsgemäße Maleinimid-funktionalisierte MTX-Derivate schnell und selektiv an HSA binden, zeigt Beispiel 2. Die Reaktion der neuen MTX-Derivate mit Serumproteinen kann auch extrakorporal durchgeführt werden, z. B. mit einer zur Infusion vorgesehenen Albumin-, Blut- oder Serummenge.

Gegenüber Methotrexat-Konjugaten mit synthetischen Polymeren als Trägersystemen besitzen die der Erfindung zugrunde liegenden MTX-PeptidDerivate des weiteren den Vorteil, chemisch eindeutig definiert zu sein.

Die Figuren zeigen:
Figur 1: Chromatogramme von Humanplasma, EMC-D-Ala-Phe-Lys-Lys(γ-MTX)-OH (3) und EMC-D-Ala-Phe-Lys-Lys(γ-MTX)-OH (3) nach 2-minütiger Inkubation mit Humanplasma bei 37 °C (Detektion bei λ= 300 nm).
Figur 2: Chromatogramme von EMC-Arg-Ala-Phe-Met-Lys(y-MTX)-OH (C162) (200 µM) nach 2-minütiger Inkubation mit Humanplasma bei 37°C und nach 5-minütiger Inkubation mit Humanplasma, das 30 min mit EMC (1000 µM) vorinkubiert wurde.
Figur 3: ein Chromatogramm des HSA-Konjugats von EMC-D-Ala-Phe-Lys-Lys(γ-MTX)-OH nach 4 h Inkubation mit humanem Plasmin (Detektion bei λ = 370 nm).
Figur 4: Chromatogramme des HSA-Konjugats von EMC-D-Ala-Phe-Lys-Lys(λ-MTX)-OH (3) nach 0, 1, 4 und 20 h Inkubation mit humanem Plasmin und nach 24 h in Puffer (Detektion bei λ = 300 nm).
Figur 5: ein Chromatogramm des HSA-Konjugats von EMC-D-Ala-Phe-Lys-Lys(γ-MTX)-OH nach 4 h Inkubation mit Cathepsin B (Detektion bei λ= 370 nm).
Figur 6: Chromatogramme des HSA-Konjugats von EMC-D-Ala-Phe-Lys-Lys(γ-MTX)-OH (3) nach 0, 1, 4 und 24 h Inkubation mit Cathepsin B und nach 24 h in Puffer (Detektion bei λ = 300 nm).
Figur 7: ein Chromatogramm des HSA-Konjugats von EMC-D-Ala-Phe-Lys-Lys(γ-MTX)-OH nach 4 h Inkubation mit OVCAR-3-Tumorhomogenat (Detektion bei λ = 370 nm).
Figur 8: ein Chromatogramm des HSA-Konjugats von EMC-Arg-Ala-Phe-Met-Lys(y-MTX)-OH (C162) nach 4 h Inkubation mit Synovialflüssigkeiten von Patienten mit RA (Detektion bei λ = 370 nm).
Figur 9: eine Grafik, die den Verlauf des Tumorwachstums in einem OVCAR-3-Modell darstellt.
Figur 10: eine Grafik, die den Verlauf der RA-Bewertung in einem Collagen-induzierten Arthritis-Modell darstellt.
Figur 11: eine Grafik, die den Verlauf des Arthritisvorkommens in einem Collagen-induzierten Arthritis-Modell bei frühem Behandlungsprotokoll (Behandlungsbeginn ab Tag 14 der Immunisierung) darstellt.
Figur 12: eine Grafik, die den Verlauf der Arthritisbewertung in einem Collagen-induzierten Arthritis-Modell bei frühem Behandlungsprotokoll (Behandlungsbeginn ab Tag 14 der Immunisierung) darstellt.
Figur 13: eine Grafik, die den Verlauf der Arthritisbewertung in einem Collagen-induzierten Arthritis-Modell bei spätem Behandlungsprotokoll (Behandlungsbeginn ab Tag 42 der Immunisierung) darstellt.
Figur 14: eine Grafik, die den Verlauf der Arthritisbewertung in einem Collagen-induzierten Arthritis-Modell bei Zwischenbehandlungsprotokoll (Behandlungsbeginn ab Tag 30 der Immunisierung) darstellt.
Figur 15: Ergebnisse der Messung von Cytokin-, Chemokin- und Enzymkonzentrationen in einem Collagen-induzierten Arthritis-Modell.

Die nachfolgenden Beispiele erläutern die vorliegende Erfindung näher.

### Beispiele

### Beispiel 1

### Herstellung von EMC-D-Ala-Lys-Lys-L(γ-MTX)-OH

Zu einer Lösung von Methotrexat-α-*tert*.-butylester (MTX-α-OtBu) (17.85 mg, 34.96 µmol) in 150 µL wasserfreiem DMF werden nacheinander DIEA (27.2 µL, 159 µmol) und HATU (13.29 mg, 34.96 µmol) gegeben. Nach 2 min Behandlung im Ultraschallbad wird die Reaktionsmischung zu einer Lösung von EMC-D-Ala-Phe-Lys(Boc)-Lys-OH (31.78 µmol) in 1.5 mL wasserfreiem DMF gegeben und 1 h lang bei Raumtemperatur gerührt. Anschließend wird die Reaktionsmischung in 100 mL Diethylether gegeben, der Niederschlag abzentrifugiert, zweimal mit Diethylether gewaschen und im Vakuum getrocknet. Zur Abspaltung der Schutzgruppen wird das Rohprodukt ohne weitere Aufreinigung 1 h lang mit 5 mL Dichlormethan/TFA 1:1 behandelt und in 100 mL Diethylether gegeben, der Niederschlag abzentrifugiert, zweimal mit Diethylether gewaschen und im Vakuum getrocknet. Nach präparativer HPLC (C18-Reverse-Phase, MeCN/Wasser 30:70, 0.1 % TFA) und Lyophilisierung wird EMC-D-Ala-Phe-Lys-Lys(γ-MTX)-OH als hellgelber Feststoff erhalten.
ESI-MS (4.0 kV, MeCN): m/z (%) 1122.3 ([M + H]⁺, 100), 1144.4 ([M + Na]⁺, 73)

### Beispiel 2

### Bindung von EMC-D-Ala-Phe-Lys-Lys(γ-MTX)-OH an HSA im Humanplasma

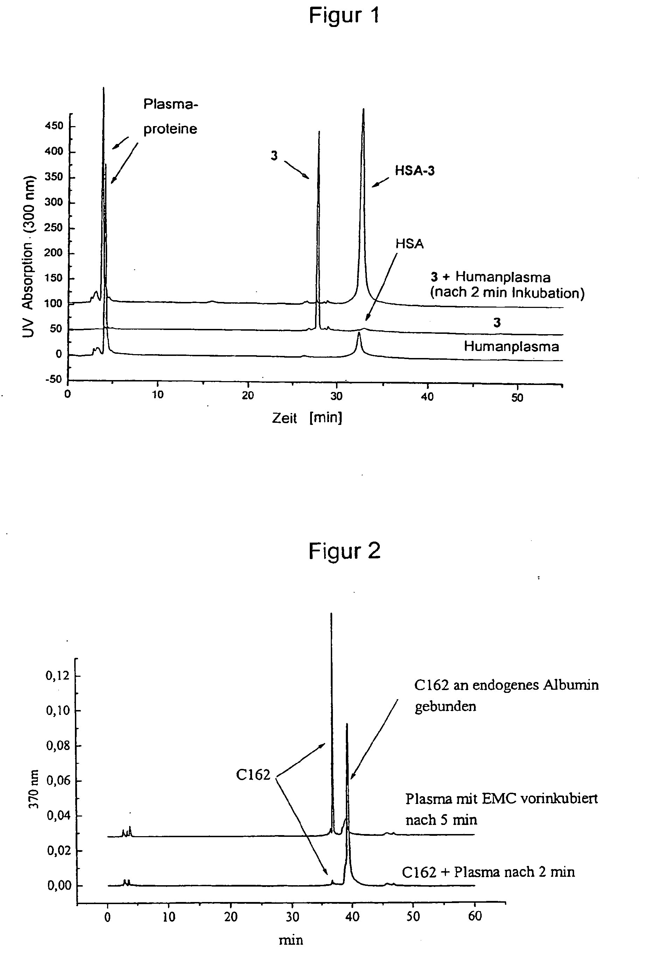

Eine Probe humanen Blutplasmas wird mit EMC-D-Ala-Phe-Lys-Lys(γ-MTX)-OH (200 µM) für einen Zeitraum von 2 min bei 37 °C inkubiert und anschließend durch Chromatographie an einer C₁₈-RP-HPLC-Säule (Symmetry^{®} 300-5 4.6 × 250 mm von Waters mit Vorsäulenfilter) durch Gradientenelution (Fluss: 1.2 mL/min; Eluent A: 30 % 20 mM K₂HPO₄ pH 7, 70 % Acetonitril; Eluent B: 85 % 20 mM K₂HPO₄ pH 7, 15 % Acetonitril; Gradient: 20 min Eluent B isokrat., 25 min 0-100 % Eluent A linear, 5 min Eluent A isokrat.) analysiert. Detektion bei der für MTX-Derivate charakteristischen Wellenlänge 300 nm zeigt eine nahezu vollständige Abnahme des Prodrug-Peaks und eine Zunahme der Absorption bei der Retentionszeit von Albumin (t - 32 min) (siehe Figur 1).

Bei einer weiteren Analyse nach 24 Stunden zeigt sich zudem anhand der entsprechenden Peakflächen, dass der Verlust an MTX weniger als 10 % beträgt.

### Beispiel 3

### Bindung von EMC-Arg-Ala-Phe-Met-Lys(y-MTX)-OH (C162) an HSA im Humanplasma

Eine Probe humanen Blutplasmas wird mit EMC-Arg-Ala-Phe-Met-Lys(y-MTX)-OH (200 µM) für einen Zeitraum von 2 min bei 37 °C inkubiert und anschließend durch Chromatographie an einer C₁₈-RP-HPLC-Säule (Symmetry^{®} 300-5 4.6 × 250 mm von Waters mit Vorsäulenfilter) durch Gradientenelution (Fluss: 1.2 mL/min; Eluent A: 30 % 20 mM K₂HPO₄ pH 7, 70 % Acetonitril; Eluent B: 85 % 20 mM K₂HPO₄ pH 7, 15 % Acetonitril; Gradient: 20 min Eluent B isokrat., 25 min 0-100 % Eluent A linear, 5 min Eluent A isokrat.) analysiert. Detektion bei der für MTX-Derivate charakteristischen Wellenlänge 370 nm zeigt eine nahezu vollständige Abnahme des Prodrug-Peaks und eine Zunahme der Absorption bei der Retentionszeit von Albumin (*t* = 40 min) (siehe Figur 2).

Eine Wiederholung des Versuchs mit humanem Blutplasma, das zuvor 5 min lang mit EMC (1000 µM) inkubiert wurde, was eine Blockierung der Cystein-34-Gruppe von Albumin zur Folge hat, zeigt bei anschließender Inkubation mit EMC-Arg-Ala-Phe-Met-Lys(y-MTX)-OH keine Bindung des Prodrugs an Albumin. Im Chromatogramm lässt sich bei 370 nm lediglich das freie Prodrug detektieren.

### Beispiel 4

### Enzymatische Spaltung des Albuminkonjugats von EMC-D-Ala-Phe-Lys-Lys(γ-MTX)-OH durch Cathepsin B und Plasmin

Herstellung des Albumin-Konjugats: 4.00 mg EMC-D-Ala-Phe-Lys-Lys(γ-MTX)-OH werden in 8 mL einer 5 %igen HSA-Lösung (Octopharm) bei Raumtemperatur gelöst und bei 37 °C 2 h lang geschüttelt. Anschließend wird die Probe durch Aufkonzentration mit Centriprep^{®}-Einmalkonzentratoren auf eine Konzentration von 700 µM gebracht.

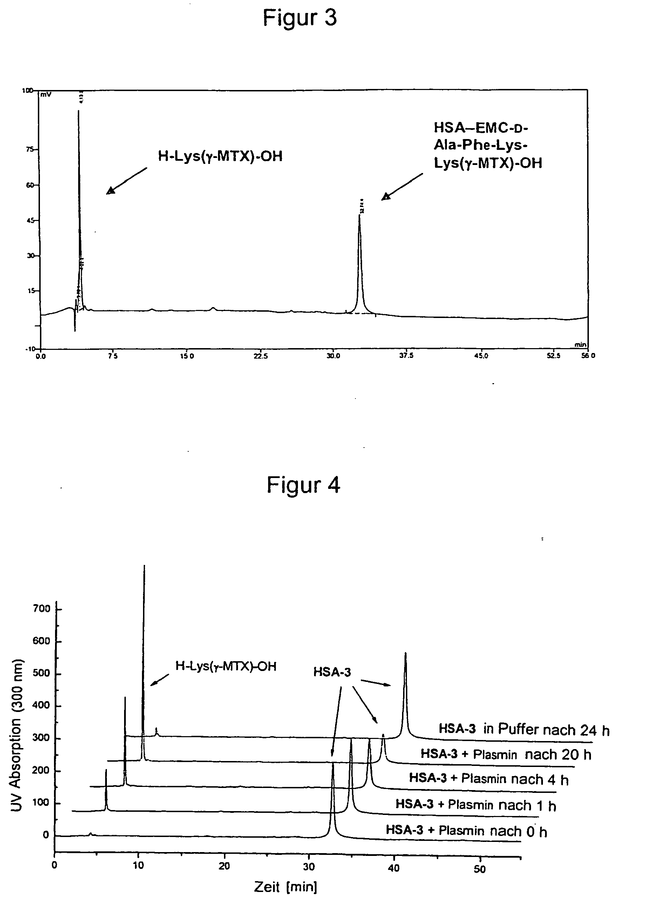

Spaltung durch Plasmin: Nun werden 100 µL der Lösung des Albumin-Konjugats mit 500 µL Puffer (4 mM Natriumphosphat, 150 mM NaCl, pH 7.4) verdünnt und 20 µL humanes Plasma-Plasmin (370 mU) zugegeben und die Mischung bei 37 °C inkubiert. Die Bestimmung der Spaltprodukte erfolgt mit der in Beispiel 2 beschriebenen HPLC-Methode (Figuren 3 und 4).

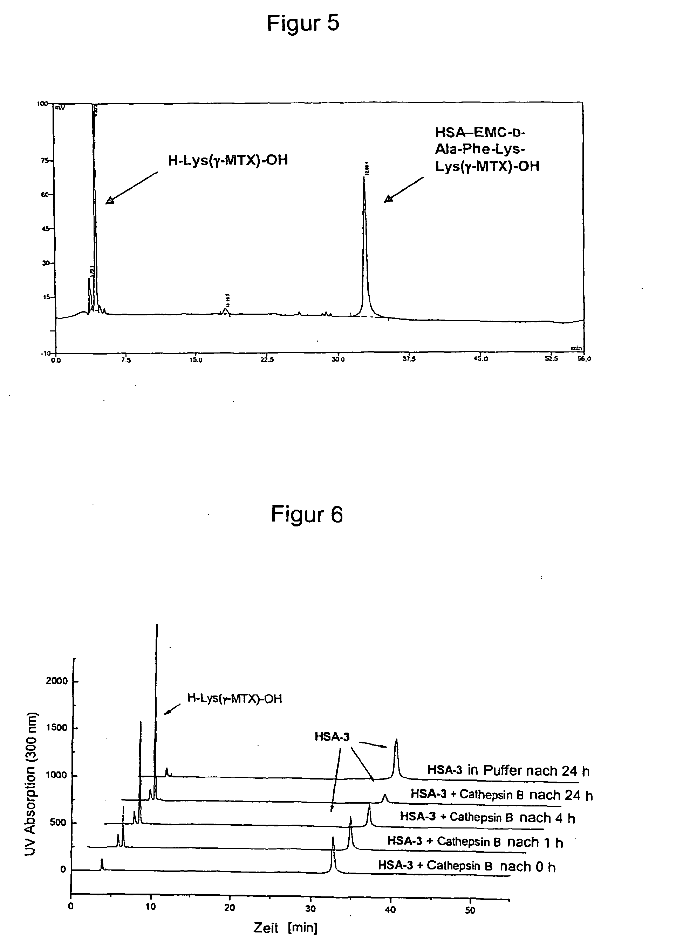

Spaltung durch Cathepsin B: Nun werden 180 µL der Lösung des Albumin-Konjugats mit 270 µL Puffer (50 mM Natriumacetat, 100 mM NaCl, 4 mM EDTA*2 Na, 8 mM L-Cystein, pH 5.0) verdünnt und 90 µL humanes Cathepsin B (2.1 U) zugegeben und bei 37 °C inkubiert. Die Bestimmung der Spaltprodukte erfolgt mit der in Beispiel 2 beschriebenen HPLC-Methode (Figuren 5 und 6).

Ergebnis: Nach jeweils ein- bzw. vierstündiger Inkubation mit den Enzymen ist in beiden Fällen die Bildung von H-Lys(γ-MTX)-OH als Spaltprodukt bei ~ 4 min erkennbar. Darüber hinaus ist ersichtlich, dass im Verlauf der Zeit die Konzentration des Albumin-Konjugats ab- und die Konzentration des Spaltprodukts zunimmt. Das Spaltprodukt ergibt sich dabei durch proteolytische Spaltung der Lys-Lys-Bindung.

### Beispiel 5

### Spaltung von HSA-EMC-D-Ala-Phe-Lys-Lys(γMTX)-OH im Homogenat eines humanen Ovarial-Xenografts (OVCAR-3)

Herstellung des Tumor-Homogenats: Das Tumormaterial wird mittels eines Skalpells stark zerkleinert und 200 mg der Masse werden mit 800 µL Puffer (Tris-Puffer pH 7.4) unter Zusatz von 3-4 Glasperlen im Schüttler homogenisiert. Anschließend wird bei 4 °C zentrifugiert und der Überstand auf 200 µL aliquotiert.

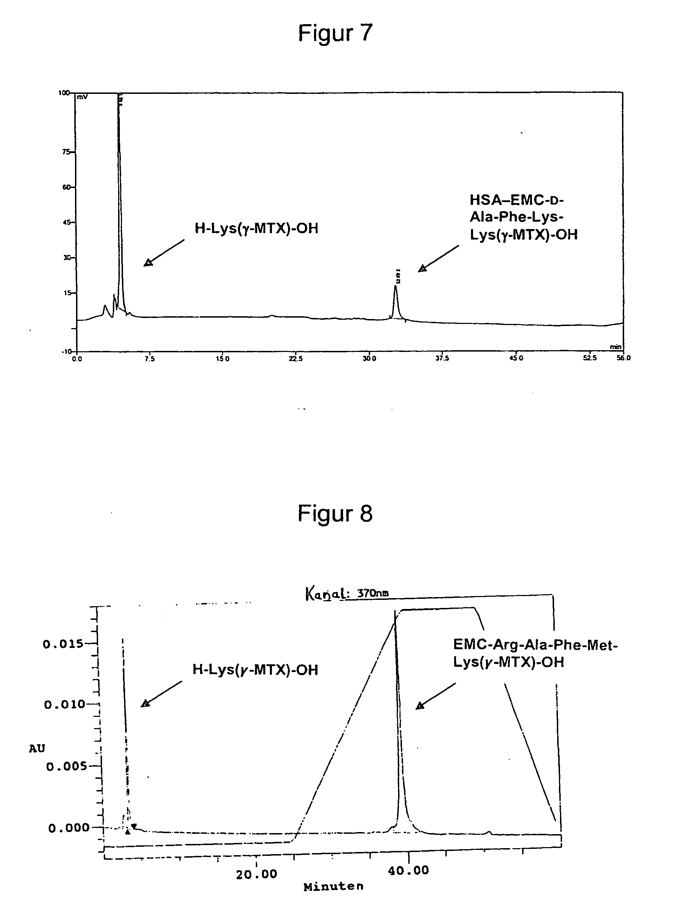

Nun werden 100 µL der in Beispiel 4 beschriebenen Lösung des Albumin-Konjugats von EMC-D-Ala-Phe-Lys-Lys(γ-MTX)-OH mit 500 µL Homogenatlösung (Homogenat, 1:2 verdünnt mit Puffer [4 mM Natriumphosphat, 150 mM NaCl, pH 7.4]) verdünnt und bei 37 °C inkubiert. Die Bestimmung der Spaltprodukte erfolgt mit der in Beispiel 2 beschriebenen HPLC-Methode (Figur 7).

Ergiebnis: Nach vierstündiger Inkubation mit OVCAR-3-Tumorhomogenat ist die Bildung von H-Lys(γ-MTX)-OH als Spaltprodukt erkennbar.

### Beispiel 6

### Spaltung von EMC-Arg-Ala-Phe-Met-Lys(y-MTX)-OH (C162) in Synovialflüssigkeiten von Patienten mit RA

Herstellung des Albumin-Konougats: 4.00 mg EMC-Arg-Ala-Phe-Met-Lys(y-MTX)-OH werden in 8 mL einer 5 %igen HSA-Lösung (Octopharm) bei Raumtemperatur gelöst und bei 37 °C 2 h lang geschüttelt. Anschließend wird die Probe durch Aufkonzentration mit Centriprep^{®}-Einmalkonzentratoren auf eine Konzentration von 700 µM gebracht.

Nun werden 70 µL der Lösung des Albumin-Konjugats von EMC-Arg-Ala-Phe-Met-Lys(y-MTX)-OH mit 140 µL Synovialflüssigkeit (Synovialflüssigkeit von sechs Patienten mit rheumatoider Arthritis, 1:1 verdünnt mit dest. Wasser) verdünnt und bei 37 °C inkubiert. Die Bestimmung der Spaltprodukte erfolgt mit der in Beispiel 2 beschriebenen HPLC-Methode (Figur 8).

Ergebnis: Nach vierstündiger Inkubation mit der Synovialflüssigkeit von Patienten mit rheumatoider Arthritis ist die Bildung von H-Lys(γ-MTX)-OH als Spaltprodukt erkennbar.

### Beispiel 7

### Wirksamkeit von EMC-D-Ala-Phe-Lys-Lys(γ-MTX)-OH und EMC-Arg-Arg-Ala-Met-Lys(γ-MTX)-OH in vivo (tumorhemmende Eigenschaften)

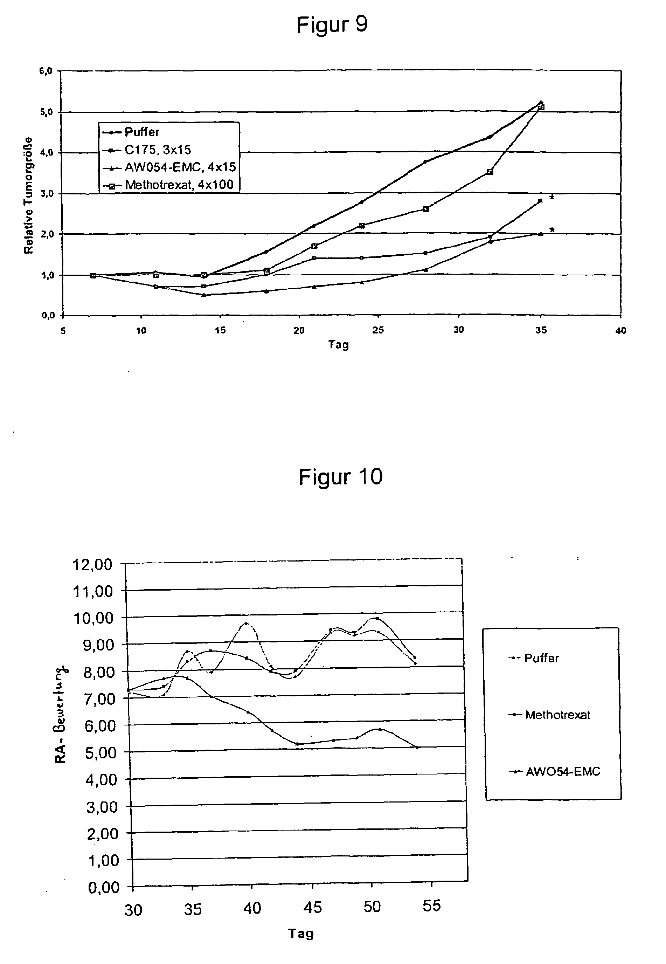

Die unten und in Figur 9 aufgeführten biologischen Daten verdeutlichen eine erhöhte in-vivo-Wirksamkeit von EMC-o-Ala-Phe-Lys-Lys(γ-MTX)-OH (AW054-EMC) und EMC-Arg-Arg-Ala-Met-Lys(y-MTX)-OH (C175) im Vergleich zu freiem Methotrexat.

Tiere: Nacktmäuse NMRI; Tumormodell: OVCAR-3 (Ovarialkarzinom subkutan wachsend)
Therapie: Tag 7, 14, 21, 28; i. v. (10 mM Natriumphosphat/ 5% D-Glucose-Puffer pH 6.4); Dosen beziehen sich auf Methotrexat-Äquivalente.

| Substanz | Dosis [mg/Kg] | Körpergewichtsänderung [%] | T/C [%] |
|---|---|---|---|
| | | | maximal |
| MTX | 4 x 100 | +19 | 69 |
| AW054-EMC | 4 x 15 | +12 | 29 |
| C175 | 3 x 15 | +7 | 40 |

### Beispiel 8

### Wirksamkeit von EMC-D-Ala-Phe-Lys-Lys(γ-MTX)-OH in vivo (antirheumatische Eigenschaften)

Die unten und in Figur 10 aufgeführten biologischen Daten verdeutlichen eine erhöhte in-vivo-Wirksamkeit von EMC-D-Ala-Phe-Lys-Lys(γ-MTX)-OH (AW054-EMC) im Vergleich zu freiem Methotrexat.

Tiere: Mäuse (m, DBA/1; Modell: Collagen-induziertes Arthritis-Modell)
Therapie: Tag 30, 34, 37, 41, 44, 48; i. v. (10 mM Natriumphosphat/ 5% _{D}-Glucose-Puffer pH 6.4); Dosen beziehen sich auf Methotrexat-Äquivalente.

| Substanz | Dosis [mg/Kg] | Körpergewichtsänderung [%] | RA-Bewertung |
|---|---|---|---|
| | | | Tag 55 |
| Kontrolle | - | +9.5 | 8.10 |
| MTX | 6 x 35 | +6.9 | 8.30 |
| AW054-EMC | 6x 20 | -0.2 | 5.00 |

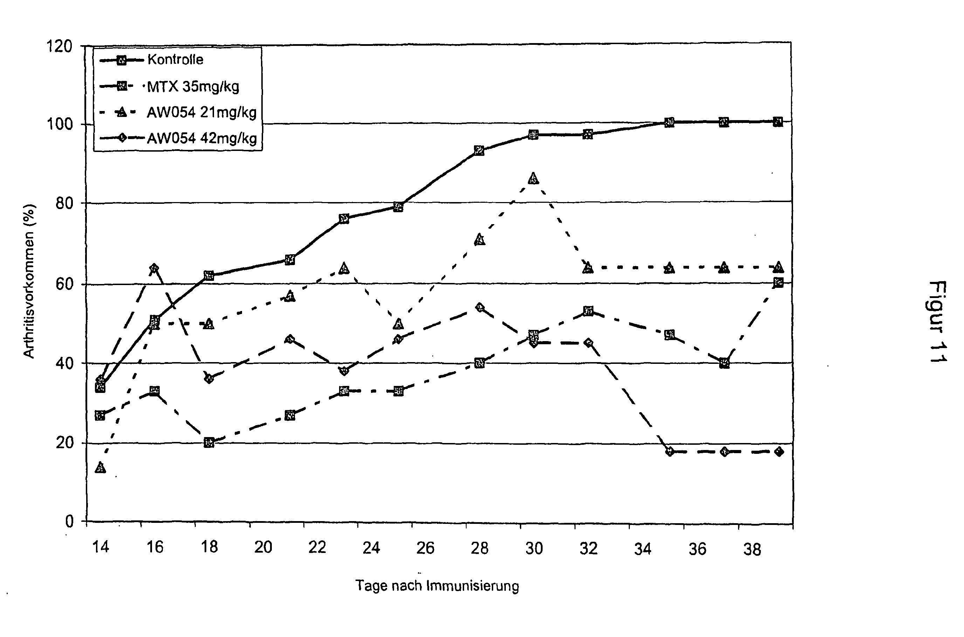

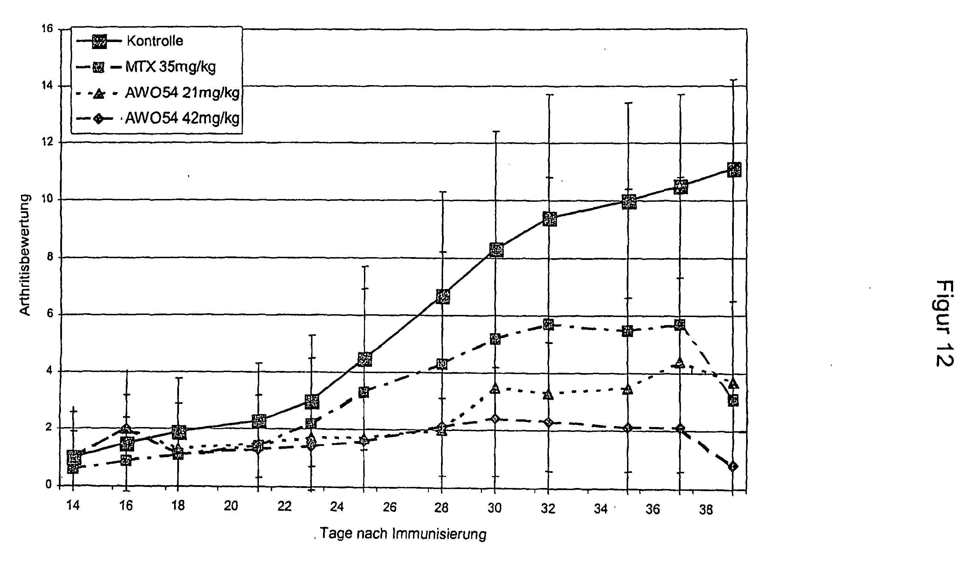

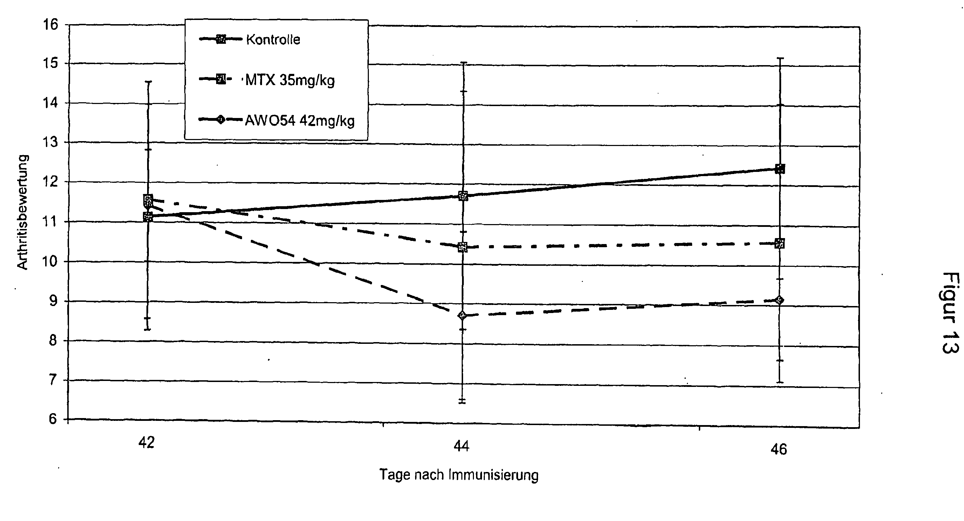

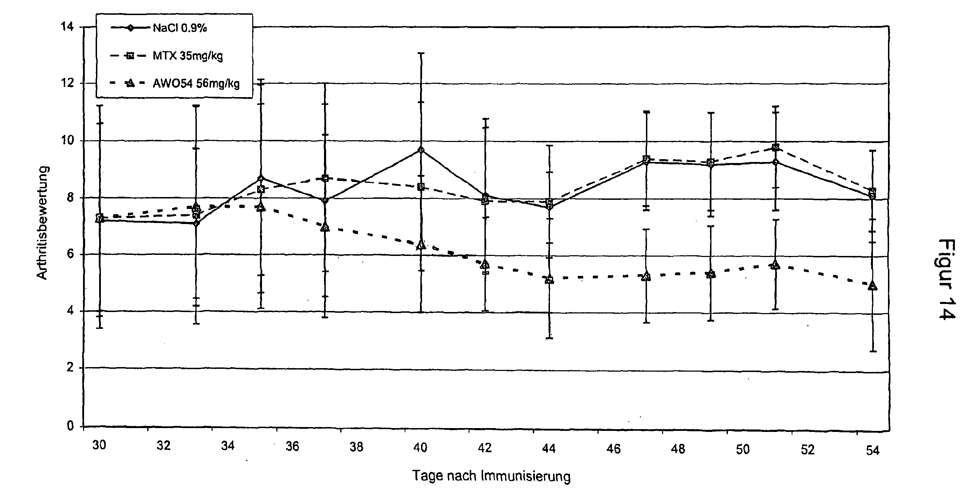

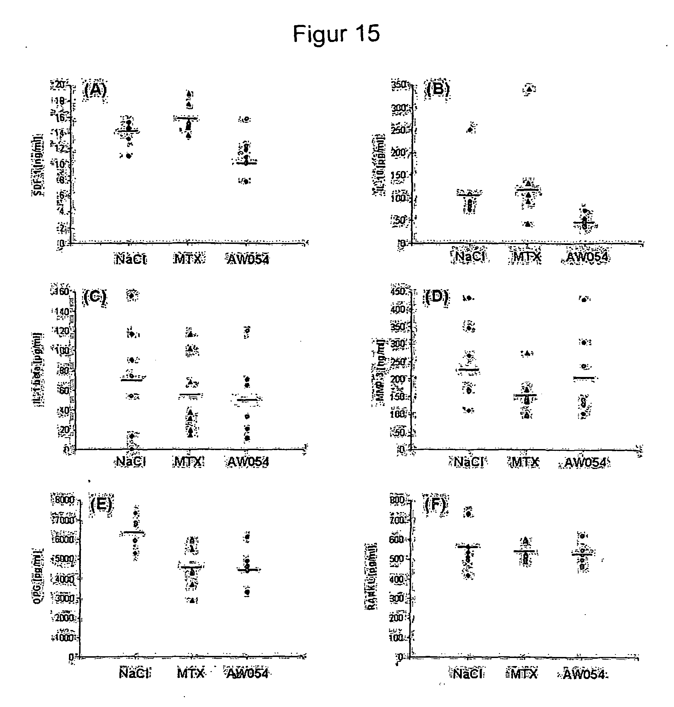

Die unten, in den Figuren 11 bis 15 und der Tabelle 1 aufgeführten biologischen Daten verdeutlichen erneut eine erhöhte in-vivo-Wirksamkeit von EMC-D-Ala-Phe-Lys-Lys(y-MTX)-OH (AW054-EMC) im Vergleich zu freiem Methotrexat.

Tiere: Mäuse (m, DBA/1; Modell: Collagen-induziertes Arthritis-Modell)
Therapie: zweimal die Woche ab Tag 14, 42 bzw. 30; i. v. (10 mM Natriumphosphat/ 5% D-Glucose-Puffer pH 6.4); Dosen beziehen sich auf Methotrexat-Äquivalente. Substanzen und Dosen sind in den Figuren 11 bis 15 angegeben.
Die Messungen der Proteinkonzentrationen im Serum nach 6-tägiger Behandlung erfolgen per Elisa (im Handel über R&D Systems Wiesbaden Deutschland erhältlich) nach dem Protokoll des Herstellers.

Die nachfolgende Tabelle 1 zeigt die Reaktion auf die Behandlung mit MTX oder verschiedenen Dosen AW054 im Vergleich mit der NaCl-Kontrolle im frühen Behandlungsprotokoll. Die Behandlung mit AW054 ergibt ein verringertes Vorkommen von ausgebildeter Arthritis am Ende des Experiments, verringert die mittlere Arthritisbewertung, verlängert den Zeitraum bis zum ersten Auftreten von Arthritis und induziert eine Verbesserung oder sogar ein Abklingen bei entwickelter Arthritis.

**Tabelle 1**

| (±Standardabweichung) | NaCl Kontrolle | MTX 35mg/kg | AW054 21 mg/kg | AW054 42mg/kg |
|---|---|---|---|---|
| | n=29 | n=15 | n=14 | n=11 |
| Arthritisvorkommen am Ende des Experiments (%) | 29 (100) | 9 (60) | 9 (64) | 2 (18) |
| Mittlere Arthritisbewertung am Ende des Experiments | 11,1 (±3,1) | 3,1 (±3,4) | 3,7 (±4,3) | 0,8 (±2,4) |
| Mittlere Zeit bis zum Ausbruch neuer Arthritis nach dem Behandlungsstart in Tagen | 8,3 (±5,7) | 17,3 (±10,8) | 6,8 (±7,6) | 11,5 (±11,1) |
| Verbessertes oder erreichtes Abklingen nach geschaffener Krankheit | 0 (0) | 8 (53) | 10(71) | 8 (73) |

Anhand der Beispiele wird ersichtlich, dass nach Inkubation mit humanem Blutplasma bereits nach 2 min das entsprechende Albumin-Konjugat im Wesentlichen gebildet ist. Die Konjugate sind ausreichend plasma-stabil, und es kann eine wirksame Spaltung in der Gegenwart sowohl von humanem Plasmin als auch Cathepsin B beobachtet werden. Die Spaltung resultiert in der Bildung von beispielsweise H-Lys(γ-MTX)-OH; welches das einzige niedermolekulare Spaltprodukt darstellt. Darüber hinaus wird dieses Spaltprodukt jedoch nicht weiter in MTX und Lysin gespalten, und die *in vivo* Experimente legen entsprechend nahe, dass das erfindungsgemäße MTX-Lysin-Derivat *per se* hochaktiv ist. Verglichen mit Methotrexat selbst zeigt es eine verstärkte Effizienz bei einer sehr viel kleineren Dosis. Beim Collagen-induzierten Arthritis-Modell liegt diese bei etwa 20 % der entsprechenden Methotrexat-Äquivalenzdosis. Darüber hinaus ist das Derivat über einen längeren Zeitraum aktiv, und die Serumkonzentrationen von beispielsweise SDF-1, OPG und IL-10 sind signifikant verringert.

## Patentansprüche

1. Methotrexat-Derivat der Formel I: 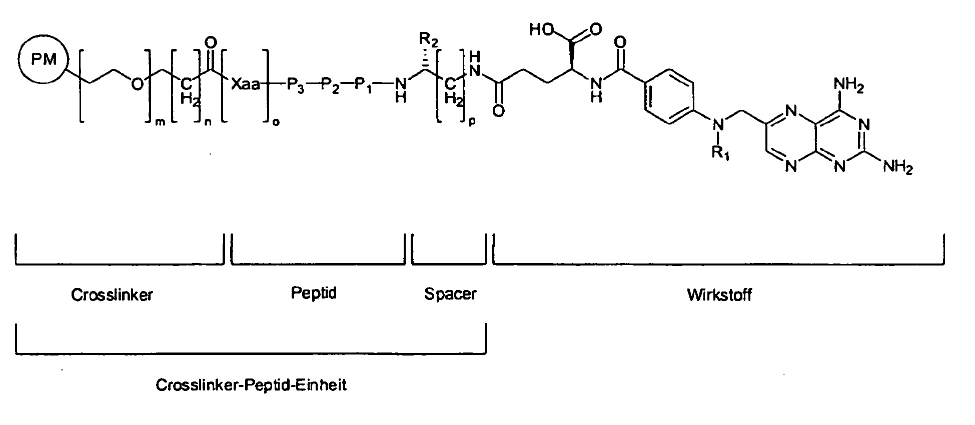 worin
R₁ = H oder CH₃
R₂ = H oder COOH
P₁ = Lysin, Methionin, Alanin, Prolin oder Glycin
P₂ = Leucin, Phenylalanin, Methionin, Alanin, Prolin oder Tyrosin
P₃ = D-Alanin, Alanin, D-Valin, Valin, Leucin oder Phenylalanin
Xₐₐ = Aminosäure mit basischer Seitenkette
m = 0 bis 6
n = 0 bis 5
o = 0 bis 2
p = 1 bis 10
PM eine proteinbindende Gruppe bedeuten.

2. Methotrexat-Derivat nach Anspruch 1, wobei PM ausgewählt ist aus der Gruppe, bestehend aus einer Maleinimidgruppe, einer 2-Dithiopyridylgruppe, einer Halogenacetamidgruppe, einer Halogenacetatgruppe, einer Disulfidgruppe, eine Acrylsäureestergruppe, eine Monoalkylmaleinsäureestergruppe, einer Monoalkylmaleaminsäureamidgruppe, einer *N*-Hydroxysuccinimidylestergruppe, einer Isothiocyanatgruppe und einer Aziridingruppe, die gegebenenfalls substituiert sein können.

3. Methotrexat-Derivat nach Anspruch 2, wobei PM eine Maleinimidgruppe ist, die gegebenenfalls substituiert sein kann.

4. Methotrexat-Derivat nach Anspruch 3, worin m = 0 und n = 4 sind.

5. Methotrexat-Derivat nach Anspruch 3, worin m = 3 und n = 1 sind.

6. Methotrexat-Derivat nach einem der vorhergehenden Ansprüche, worin R₁ = CH₃ ist.

7. Methotrexat-Derivat nach einem der vorhergehenden Ansprüche, worin R₂ = COOH und p = 4 ist.

8. Methotrexat-Derivat nach einem der vorhergehenden Ansprüche, worin P₁ = Lysin, Alanin oder Methionin ist.

9. Methotrexat-Derivat nach einem der vorhergehenden Ansprüche, worin P₂ = Phenylalanin, Methionin, Alanin oder Tyrosin ist.

10. Methotrexat-Derivat nach einem der vorhergehenden Ansprüche, worin P₃ = D-Alanin, Alanin, D-Valin, Valin oder Phenylalanin ist.

11. Methotrexat-Derivat nach einem der Ansprüche 8 bis 10, worin P₁ = Lysin, P₂ = Leucin oder Phenylalanin und P₃ = Alanin, D-Alanin, Valin oder D-Valin sind.

12. Methotrexat-Derivat nach Anspruch 11, worin P₂ = Leucin und P₃ = D-Valin sind.

13. Methotrexat-Derivat nach Anspruch 11, worin P₂ = Leucin und P₃ = Valin sind.

14. Methotrexat-Derivat nach Anspruch 11, worin P₂ = Phenylalanin und P₃ = D-Alanin sind.

15. Methotrexat-Derivat nach Anspruch 11, worin P₂ = Phenylalanin und P₃ = Alanin sind.

16. Methotrexat-Derivat nach einem der Ansprüche 8 bis 10, worin P₁ = Methionin, P₂ = Methionin, Alanin oder Phenylalanin und P₃ = Alanin oder Phenylalanin sind.

17. Methotrexat-Derivat nach Anspruch 16, worin P₂ = Alanin und P₃ = Phenylalanin sind.

18. Methotrexat-Derivat nach Anspruch 16, worin P₂ = Phenylalanin und P₃ = Alanin sind.

19. Methotrexat-Derivat nach Anspruch 16, worin P₂ = Methionin und P₃ = Alanin sind.

20. Methotrexat-Derivat nach Anspruch 16, worin P₂ = Methionin und P₃ = Phenylalanin sind.

21. Methotrexat-Derivat nach einem der vorhergehenden Ansprüche, worin o = 0 ist.

22. Methotrexat-Derivat nach einem der vorhergehenden Ansprüche, worin Xₐₐ = Arginin, Lysin oder Histidin bedeutet.

23. Methotrexat-Derivat nach Anspruch 22, worin Xₐₐ = Arginin und o = 2 bedeuten.

24. Verfahren zur Herstellung von Methotrexat-Derivaten nach einem der vorhergehenden Ansprüche, wobei ein Methotrexat-Derivat der allgemeinen Formel II 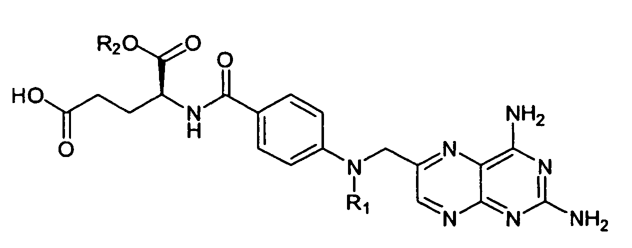 worin
R₁ = CH₃, H oder COCF₃
R₂ = C(CH₃)₃, eine Alkoxy-substituierte Benzylgruppe oder eine Trialkylsilylgruppe
bedeuten, mit einer Crosslinker-Peptid-Einheit der allgemeinen Formel III 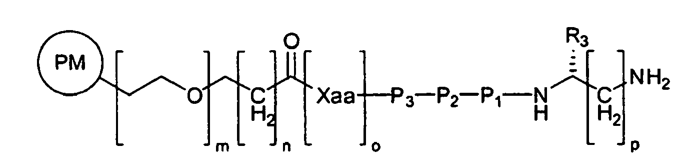 worin
R₃ = H, COOH oder COOtBu
P₁ = Lysin, Methionin, Alanin, Prolin oder Glycin
P₂ = Leucin, Phenylalanin, Methionin, Alanin, Prolin oder Tyrosin
P₃ = D-Alanin, Alanin, D-Valin, Valin, Leucin oder Phenylalanin
Xₐₐ = Aminosäure mit basischer Seitenkette
m = 0 bis 6
n = 0 bis 5
o = 0 bis 2
p = 1 bis 10
PM eine proteinbindende Gruppe
bedeuten, wobei nukleophile Gruppen an P₁, P₂ und Xaa gegebenenfalls durch geeignete Schutzgruppen geschützt vorliegen, in Gegenwart eines Carbonsäure-Aktivierungsreagenz und unter Zusatz von Katalysatoren/Hilfsbasen umgesetzt und in einem zweiten Schritt mit einer Säure, gegebenenfalls unter Zusatz von Kationenabfangreagenzien, behandelt wird.

25. Verfahren nach Anspruch 24, wobei das Carbonsäure-Aktivierungsreagenz ausgewählt ist aus der Gruppe, bestehend aus *N,N'-*Diiopropylcarbodiid, *N*,*N*'-Dicyclohexylcarbodiimid, (Benzotriazol-1-yloxy)tris(dimethylamino)phosphonium-Hexafluorophosphat, 2-Chlor-1-methylpyridiniumiodid und *O*-(Azabenzotriazol-1-yl)-*N*,*N*,*N*',*N*'-tetramethyluronium-Hexafluorophosphat.

26. Verfahren nach Anspruch 24, wobei der Katalysator/Hilfsbase ausgewählt ist aus der Gruppe, bestehend aus Trialkylaminen, Pyridin, 4-Dimethylaminopyridin (DMAP) und Hydroxybenzotriazol (HOBt), oder einer Kombination davon.

27. Verfahren nach einem der Ansprüche 24 bis 26, wobei als Carbonsäure-Aktivierungsreagenz *O*-(Azabenzotriazol-1-yl)-*N*,*N*,*N*',*N*'-tetramethyluronium-Hexafluorophosphat in Verbindung mit *N*-Ethyldiisopropylamin verwendet wird.

28. Verfahren nach Anspruch 24, wobei als Säure im zweiten Schritt Chlorwasserstoff eingesetzt wird.

29. Verfahren nach Anspruch 24, wobei als Säure im zweiten Schritt Trifluoressigsäure eingesetzt wird.

30. Verfahren nach Anspruch 24, wobei im zweiten Schritt das Kationenabfangreagenz ausgewählt ist aus der Gruppe, bestehend aus Wasser, Phenol, Thioanisol, Diiopropylsilan und 1,2-Ethandithiol, oder einer Kombination davon.

31. Verfahren gemäß Anspruch 24, wobei Methotrexat-α-*tert*.-butylester mit ((((6-Maleinimidohexanoyl)D-alanyl)phenylalanyl)*tert*.-butoxylcarbonyllysyl) lysin-Trifluoracetat unter Einsatz von *O*-(Azabenzotriazol-1-yl)-*N*,*N*,*N*',*N*'-tetramethyluronium-Hexafluorophosphat in Verbindung mit *N-*Ethyldüsopropylamin umgesetzt und in einem zweiten Schritt mit Trifluoressigsäure behandelt wird.

32. Arzneimittel, umfassend ein Methotrexat-Derivat nach einem der Ansprüche 1 bis 23, gegebenenfalls zusammen mit einem oder mehreren pharmazeutisch verträglichen Hilfsstoffen.

33. Arzneimittel nach Anspruch 32 zur Behandlung von Krebskrankheiten.

34. Arzneimittel nach Anspruch 32 zur Behandlung von rheumatischen Erkrankungen.

## Claims

1. A methotrexate derivative of the structural formula I: 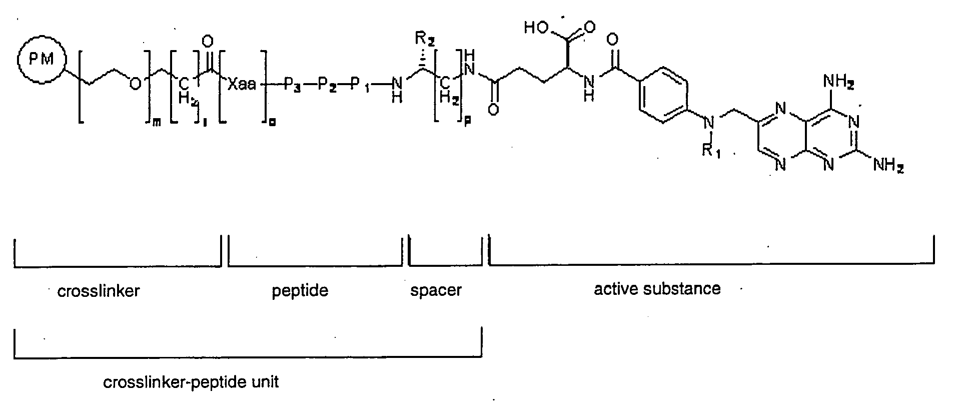 wherein
R₁ = H or CH₃
R₂ = H or COOH
P₁ = lysine, methionine, alanine, proline or glycine
P₂ = leucine, phenylalanine, methionine, alanine, proline or tyrosine
P₃ = D-alanine, alanine, D-valine, valine, leucine or phenylalanine
Xₐₐ = amino acid with alkaline side chain
m = 0 to 6
n = 0 to 5
o=0 to 2
p=1 to 10
PM is a protein-binding group.

2. The methotrexate derivative according to claim 1, wherein PM is selected from the group consisting of a maleinimide group, a 2-dithiopyridyl group, a halogen acetamide group, a halogen acetate group, a disulphide group, an acrylic acid ester group, a monoalkyl maleic acid ester group, a monoalkyl maleamine acid amide group, an *N*-hydroxy succinimidyl ester group, an isothiocyanate group and an aziridine group, which may be optionally substituted.

3. The methotrexate derivative according to claim 2, wherein PM is a maleinimide group, which may be optionally substituted.

4. The methotrexate derivative according to claim 3, wherein m = 0 and n = 4.

5. The methotrexate derivative according to claim 3, wherein m = 3 and n = 1.

6. The methotrexate derivative according to any one of the preceding claims, wherein R₁ = CH₃.

7. The methotrexate derivative according to any one of the preceding claims, wherein R₂ = COOH and p = 4.

8. The methotrexate derivative according to any one of the preceding claims, wherein P₁ = lysine, alanine or methionine.

9. The methotrexate derivative according to any one of the preceding claims, wherein P₂ = phenylalanine, methionine, alanine or tyrosine.

10. The methotrexate derivative according to any one of the preceding claims, wherein P₃ = D-alanine, alanine, D-valine, valine or phenylalanine.

11. The methotrexate derivative according to any one of claims 8 to 10, wherein P₁ = lysine, P₂ = leucine or phenylalanine and P₃ = alanine, D-alanine, valine or D-valine.

12. The methotrexate derivative according to claim 11, wherein P₂ = leucine and P₃ = D-valine.

13. The methotrexate derivative according to claim 11, wherein P₂ = leucine and P₃ = valine.

14. The methotrexate derivative according to claim 11, wherein P₂ = phenylalanine and P₃ = D-alanine.

15. The methotrexate derivative according to claim 11, wherein P₂ = phenylalanine and P₃ = alanine.

16. The methotrexate derivative according to any one of claims 8 to 10, wherein P₁ = methionine, P₂ = methionine, alanine or phenylalanine and P₃ = alanine or phenylalanine.

17. The methotrexate derivative according to claim 16, wherein P₂ = alanine and P₃ = phenylalanine.

18. The methotrexate derivative according to claim 16, wherein P₂ = phenylalanine and P₃ = alanine.

19. The methotrexate derivative according to claim 16, wherein P₂ = methionine and P₃ = alanine.

20. The methotrexate derivative according to claim 16, wherein P₂ = methionine and P₃ = phenylalanine.

21. The methotrexate derivative according to any one of the preceding claims, wherein o = 0.

22. The methotrexate derivative according to any one of the preceding claims, wherein Xₐₐ = arginine, lysine or histidine.

23. The methotrexate derivative according to claim 22, wherein Xₐₐ = arginine and o = 2.

24. A method for producing methotrexate derivatives according to any one of the preceding claims, wherein a methotrexate derivative having the general structural formula II 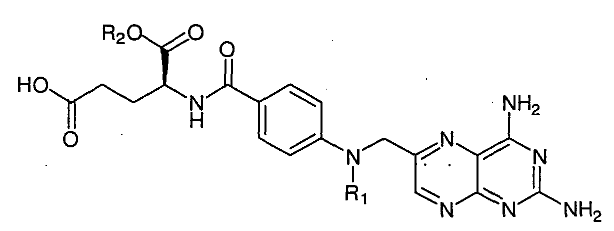 wherein
R₁ = CH₃, H or COCF₃
R₂ = C(CH₃)₃, an alkoxy-substituted benzyl group or a trialkyl silyl group,
is reacted with a crosslinker-peptide unit of the general structural formula III 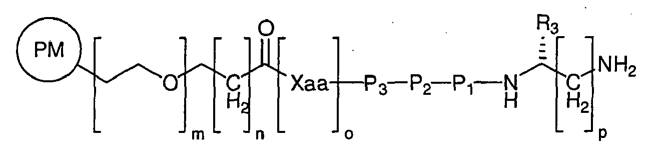 wherein
R₃ = H, COOH or COOtBu
P₁ = lysine, methionine, alanine, proline or glycine
P₂ = leucine, phenylalanine, methionine, alanine, proline or tyrosine
P₃ = D-alanine, alanine, D-valine, valine, leucine or phenylalanine
Xₐₐ = amino acid with alkaline side chain
m = 0 to 6
n = 0 to 5
o = 0 to 2
p = 1 to 10
PM is a protein-binding group,
wherein nucleophilic groups are present at P₁, P₂ and Xaa, optionally protected by suitable protective groups,
in the presence of a carboxylic acid activation reagent and under addition of catalysts/auxiliary bases, and treated in a second step with an acid, optionally with addition of cation-scavenging reagents.

25. The method according to claim 24, wherein the carboxylic acid activation reagent is selected from the group consisting of *N*,*N*'-diisopropyl carbodiimide, *N*,*N*'-dicyclohexyl carbodiimide, (benzotriazol-1-yloxy)tris(dimethylamino)phosphonium hexafluorophosphate, 2-chloro-1-methylpyridinium iodide and *O*-(azabenzotriazol-1-yl)-*N*,*N*,*N*',*N*'-tetramethyluronium hexafluorophosphate.

26. The method according to claim 24, wherein the catalyst/auxiliary base is selected from the group consisting of trialkylamines, pyridine, 4-dimethylaminopyridine (DMAP) and hydroxybenzotriazole (HOBt), or a combination thereof.

27. The method according to any one of claims 24 to 26, wherein *O-*(azabenzotriazol-1-yl)-*N*,*N*,*N*',*N*'tetramethyluronium hexafluorophosphate in connection with *N*-ethyldiisopropylamine is used as carboxylic acid activation reagent.

28. The method according to claim 24, wherein hydrogen chloride is used as an acid in the second step.

29. The method according to claim 24, wherein trifluoroacetic acid is used as an acid in the second step.

30. The method according to claim 24, wherein in the second step, the cation-scavenging reagent is selected from the group consisting of water, phenol, thioanisole, diisopropylsilane and 1,2-ethane dithiole, or a combination thereof.

31. The method according to claim 24, wherein methotrexate-α-*tert*-butylester is reacted with ((((6-maleinimidohexanoyl)D-alanyl)phenylalanyl)tert.-butoxylcarbonyllysyl) lysine-trifluoreacetate using *O*-(azabenzotriazol-1-yl)-*N*,*N*,*N*'*N*'-tetramethyluronium hexafluorophosphate in connection *N-*ethyldiisopropylamine and treated with trifluoroacetic acid in a second step.

32. A medicament comprising a methotrexate derivative according to any one of claims 1 to 23, optionally together with one or more pharmaceutically acceptable auxiliary agents.

33. The medicament according to claim 32 for the treatment of cancer diseases.

34. The medicament according to claim 32 for the treatment of rheumatic diseases.

## Revendications

1. Dérivé de méthotrexate de formule I : 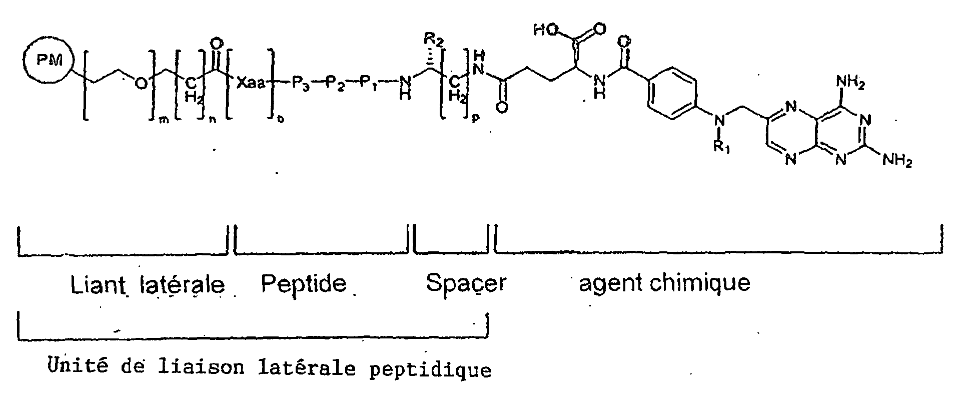 dans lequel
R₁ = H ou CH₃
R₂ = H ou COOH
P₁ = lysine, méthionine, alanine, proline ou tyrosine
P₂ = leucine, phénylalanine, méthionine, alanine, proline ou tyrosine
P₃ = _{D}-alanine, alanine, _{D}-valine, valine, leucine ou phénylalanine
Xₐₐ = acide aminé avec chaîne latérale basique
m = 0 à 6
n = 0 à 5
o = 0 à 2
p = 1 à 10
PM signifient un groupe de liaison protéique

2. Dérivé de méthotrexate selon la revendication 1, sachant que PM est choisi dans le groupe constitué d'un groupe de maléinimide, d'un groupe de 2-dithiopyridyle, d'un groupe d'acétamides halogènés, d'un groupe d'acétates halogénés, d'un groupe de disulfure, d'un groupe d'esters d'acides acryliques, d'un groupe d'esters mono-alkyliques d'acide maléique, d'un groupe d'amides mono-alkyliques d'acide maléamine, d'un groupe d'esters de N hydroxysuccinimidyle , d'un groupe d'isothiocyanate et d'un groupe d'aziridine, qui le cas échéant peuvent être substitués.

3. Dérivé de méthotrexate selon la revendication 2, sachant que PM est un groupe de maléinimide qui peut être substitué le cas échéant.

4. Dérivé de méthotrexate selon la revendication 3, dans lequel sont m = 0 et n = 4.

5. Dérivé de méthotrexate selon la revendication 3, dans lequel sont m=3 et n = 1.

6. Dérivé de méthotrexate selon l'une des revendications précédentes, dans lequel est R₁ = CH₃.

7. Dérivé de méthotrexate selon l'une des revendications précédentes, dans lequel est R₂ = COOH et p = 4.

8. Dérivé de méthotrexate selon l'une des revendications précédentes, dans lequel est P₁ = lysine, alanine ou méthionine.

9. Dérivé de méthotrexate selon l'une des revendications précédentes, dans lequel est P₂ = phénylalanine, méthionine, alanine ou tyrosine

10. Dérivé de méthotrexate selon l'une des revendications précédentes, dans lequel est P₃ =_{D}-alanine, alanine, _{D}-valine, valine ou phénylalanine.

11. Dérivé de méthotrexate selon l'une des revendications 8 à 10, dans lequel sont P₁ = lysine, P₂ = leucine ou phénylalanine et P₃ = alanine, _{D}-alanine, valine ou _{D}-valine.

12. Dérivé de méthotrexate selon la revendication 11, dans lequel sont P₂ = leucine et P₃ = _{D}-valine

13. Dérivé de méthotrexate selon la revendication 11, dans lequel sont P₂ = leucine et P₃ = valine.

14. Dérivé de méthotrexate selon la revendication 11, dans lequel sont P₂ = phénylalanine et P₃ = D-alanine.

15. Dérivé de méthotrexate selon la revendication 11, dans lequel sont P₂ = phénylalanine et P₃= alanine

16. Dérivé de méthotrexate selon l'une des revendications 8 à 10, dans lequel sont P₁ = méthionine, P₂ = méthionine, alanine ou phénylalanine et P₃ = alanine ou phénylalanine.

17. Dérivé de méthotrexate selon la revendication 16, dans lequel sont P₂ = alanine et P₃ = phénylalanine.

18. Dérivé de méthotrexate selon la revendication 16, dans lequel sont P₂ = phénylalanine et P₃ = alanine.

19. Dérivé de méthotrexate selon la revendication 16, dans lequel sont P₂ = méthionine et P₃= alanine.

20. Dérivé de méthotrexate selon la revendication 16, dans lequel sont P₂ = méthionine et P₃= phénylalanine.

21. Dérivé de méthotrexate selon l'une des revendications précédentes, dans lequel est 0 = 0.

22. Dérivé de méthotrexate selon l'une des revendications précédentes, dans lequel Xₐₐ signifie = arginine, lysine ou histidine.

23. Dérivé de méthotrexate selon la revendication 22, dans lequel Xₐₐ signifie = arginine et 0 signifie = 2.

24. Procédé de fabrication de dérivés de méthotrexate selon l'une des revendications précédentes, sachant qu'un dérivé de méthotrexate de formule générique II 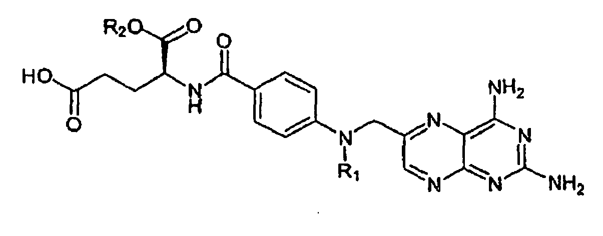 où
R₁ = CH₃, H ou COCF₃, R₂ = C(CH₃)₃ signifient un groupe de benzyle de substitution alkoxyde ou un groupe de trialkylsilyles, avec une unité de liaison latérale peptidique de formule générique III 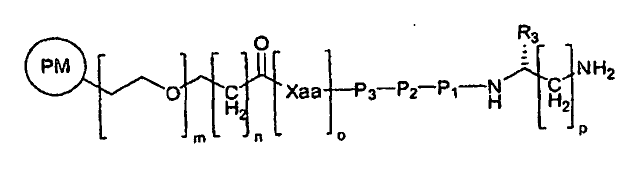 où
R₃ = H, COOH ou COOtBu
P₁ = lysine, méthionine, alanine, proline ou tyrosine
P₂ = leucine, phénylalanine, méthionine, alanine, proline ou tyrosine
P₃ = _{D}-alanine, alanine, _{D}-valine, valine, leucine ou phénylalanine
Xₐₐ = acide aminé avec chaîne latérale basique
m = 0 à 6
n = 0 à 5
o = 0 à 2
p = 1 à 10
PM signifient un groupe de liaison protéique, sachant que les groupes nucléophiles sont présents sur P₁, P₂ et Xₐₐ protégés le cas échéant par des groupes de protection appropriés, est transformé en présence d'une réactifs d'activation d'acide carboxylique et avec ajout de catalyseurs/bases auxiliaires et traité dans une deuxième étape avec un acide, le cas échéant avec supplément de réactifs de capture de cations.

25. Procédé selon la revendication 24, sachant que le réactif d'activation d'acide carboxylique est choisi dans le groupe constitué de *N*,*N*' carbodiimide diisopropyle, de *N*-*N*' carbodiimide dycyclohexyle, d'hexafluorophosphate (benzotriazol-1-yloxyde)tris(diméthylamino)phosphonium, de 2-chlore-1-méthyloroniumiodide et d'hexafluorophosphate O-(azabenzotriazol-1-yl)-*N*,*N,N*',*N*'-tétraméthyluronium.

26. Procédé selon la revendication 24, sachant que le catalyseur/la base auxiliaire est choisi dans le groupe composé de trialkylamines, de pyridine, de 4-diméthylaminopyridine (DMAP) et d'hydroxybenzotriazol (HOBt), ou d'une combinaison de ceux-ci.

27. Procédés selon l'une des revendications 24 à 26, sachant qu'en guise de réactif d'activation d'acide carboxylique on utilise de l'hexafluorophosphate *O*-(azabenzotriazol-1-yl)-*N*,*N*,*N*',*N*'-tétraméthyloronium en association avec du *N*-éthyldilsopropylamine.

28. Procédé selon la revendication 24, sachant qu'en guise d'acide on utilise du chlorure d'hydrogène dans la deuxième étape.

29. Procédé selon la revendication 24, sachant qu'en guise d'acide on utilise de l'acide acétique trifluor dans la deuxième étape.

30. Procédé selon la revendication 24, sachant que dans la deuxième étape, le réactif de capture de cations est choisi dans le groupe constitué d'eau, de phénol, de thioanisole, de diisopropylsitane et de 1,2-éthandithiole ou d'une combinaison de ceux-ci.

31. Procédé conforme à la revendication 24, sachant que l'ester de butyle tert-α-méthotrexate est transformé avec du trifluoracétate de lysine ((((6-maléinimidohexanoyl)D-alanyl)phénylalanyle)tert.-butoxylcarbonyilysyte) avec l'utilisation d'hexalfuorophosphate O-(azabenzotriazol-1-yl)-N,N,N',N'-tétraméthyluronium en association avec du N-éthyldiisopropylamine et est traité dans une deuxième étape avec de l'acide acétique trifluor.

32. Médicament, comportant un dérivé de méthotrexate selon l'une des revendications 1 à 23, le cas échéant avec une ou plusieurs substances auxiliaires pharmaceutiquement tolérées.

33. Médicament selon la revendication 32, pour le traitement de maladies cancéreuses.

34. Médicament selon la revendication 32, pour le traitement de maladies rhumatismales.
